# EUROPEAN PATENT SPECIFICATION

(11) **EP 0 609 961 B1**
(45) Date of publication and mention of the grant of the patent: **24.06.1998**
(21) Application number: 94200469.8
(22) Date of filing: 05.01.1990
(51) Int. Cl.: A61K 31/485, A61K 9/52, A61K 9/54

(54) **Sustained release pharmaceutical composition**
Arzneimittel mit verzögerter Freigabe
Composition pharmaceutique à libération retardée

(30) Priority: 06.01.1989 AU PJ2192/89
(43) Date of publication of application: 10.08.1994
(62) Divisional of application: 90300130.3
(73) Proprietor: F.H. FAULDING & CO. LIMITED, Underdale, South Australia 5032 (AU)
(72) Inventor: Morella, Angelo Mario, Campbelltown, South Australia 5014 (AU); Fisher, Mark Christopher, Woodcroft South Australia 5162 (AU)
(74) Representative: Woods, Geoffrey Corlett

(56) References cited:
- EP-A- 0 164 959
- EP-A- 0 205 282
- EP-A- 0 260 236
- EP-A- 0 327 295

## Description

The present invention relates to a sustained release pharmaceutical composition, in particular a sustained release pharmaceutical composition including an active ingredient which is an opiate agonist.

As is known in the prior art, it is desirable in the treatment of a number of diseases, both therapeutically and prophylactically to provide the active pharmaceutical ingredient in a sustained release form. Desirably the sustained release provides a generally constant rate of release over an extended period. Whilst there is known in the prior art numerous sustained release formulations, the extension of sustained release regimens to active pharmaceutical ingredients of high solubility in water has been extremely limited. It has been found in the prior art that the high solubility in water of the active ingredient tends to generate a product which is susceptible to the phenomenon known as "dose dumping". That is, release of the active ingredient is delayed for a time but once release begins the rate of release is very high. Moreover, fluctuations tend to occur in the plasma concentrations of active ingredient which increases the likelihood of toxicity. Further, some degree of diurnal variation in plasma concentration of active ingredient has also been noted.

Prior art preparations may also suffer from other disadvantages, for example bioavailability of prior art preparations may be compromised by food. This is important since complex dosage regimens may lead to non-compliance.

For example, typical highly water soluble active ingredients include the opioid drugs which still play a major role in the treatment of acute and chronic pain, particularly pain associated with terminal diseases such as cancer.

Morphine is regarded as the opioid drug of choice in the treatment of cancer pain. It is universally acknowledged that the oral route of administration is preferred if sufficient pain relief can be obtained with an acceptable profile of side effects with respect to incidence and severity. Until recently, the liquid or immediate release tablet formulations of morphine were the only dosage forms available to physicians for oral administration in the treatment of cancer pain.

The oral administration of morphine has had many critics in the prior art who point to a supposed lack of efficacy. However, the accumulated evidence, particularly from the hospice environment, indicates that this criticism is unfounded if the dose and dosing interval are specifically optimised for each patient, the morphine doses are administered before the pain returns and in a strictly regular regimen. In practical terms, this means morphine doses ranging from 10 mg to in excess of 500 mg with dosing intervals ranging from every 2 to 6 hours. A relationship between blood morphine concentration and pain relief has been established in the treatment of post-operative and cancer pain.

The studies propose that there is a minimum effective concentration (MEC) for morphine for each patient. There is a five-fold interpatient variation in MEC in the treatment of post-operative pain and an even greater variation for cancer pain. This concept of a MEC for opioids has also been demonstrated for pethidine, methadone, fentanyl and ketobemidone. Repeated oral or parenteral doses produce fluctuating blood opioid concentrations, with the peak concentrations sometimes resulting in side effects, while the trough concentrations are usually associated with inadequate pain relief. Therefore, a formulation of morphine which reduces the fluctuations in blood opioid concentrations and has a longer duration of pain relief (e.g. a sustained release preparation) has widespread potential to improve pain relief in terminal care.

Currently, there is only one such preparation (MST Continus or MS Contin) being marketed world-wide. However, the combined pharmacokinetic and pharmacodynamic data suggest that this product is actually a delayed release formulation with some sustained release characteristics. While the manufacturers recommend a 12 hour dosing interval, extensive clinical experience suggests that an 8 hour interval is more realistic for continuous pain control.

Accordingly, it is an object of the present invention to overcome, or at least alleviate, one or more of the difficulties related to the prior art.

Accordingly, in a first aspect of the present invention there is provided a sustained release pharmaceutical pellet composition including
a core element including at least one active ingredient which is an opiate agonist selected from the group consisting of the salts of codeine, dextromoramide, hydrocodone, hydromorphine, pethidine, methadone, morphine and propoxyphene, and
a core coating for the core element which is partially soluble at a highly acidic pH, and including
   a) at least 35% by weight of a matrix polymer which is insoluble independent of pH;
   b) from 1 to 60% of an acid-soluble compound at a pH of from 1 to 4, sufficient to provide a slow rate of release of the active ingredient in the stomach;
   c) from 1 to 30% of an enteric polymer which is substantially insoluble at a pH of 1 to 4 but which is soluble at a pH of from 6 to 7.5; and the components in said coating being effective such that the active ingredient is available for absorption at a relatively constant rate of release in the intestine such that the composition delivers a therapeutically effective amount of said active ingredient over the course of a predetermined interval, so as to maintain an active ingredient blood level at steady state of at least 75% of maximum blood lever (t>0.75Cₘₐₓ) for approximately 3.5 hours or greater and so that the time at which the active ingredient reaches it maximum concentration (tₘₐₓ) is 4.5 hours or greater.

By "sustained release" as used herein we mean release of active ingredient at such a rate that blood levels are maintained within the therapeutic range but below toxic levels over an extended period of time e.g. 10 to 24 hours or greater. The active ingredients typically have an aqueous solubility of approximately 1 in 30 or above.

By "bioavailability" as used herein we mean the extent to which the active ingredient is absorbed from the drug product and becomes available at the site of drug action.

The active ingredient is an opiate agonist selected from the salts of codeine, dextromoramide, hydrocodone, hydromorphine, pethidine, methadone, morphine and propoxyphene. The invention is applicable to active ingredients of high solubility whether the solubility characteristics are pH dependent or pH independent.

Examples of the solubility of active ingredients are set out in the table below.

| DRUG | SOLUBILITY (AQUEOUS) | pKA |
|---|---|---|
| Opiate Agonists | | |
| Codeine HCl | 1 in 30 | 8.2 |
| Codeine phosphate | 1 in 4 | 8.2 |
| Codeine sulphate | 1 in 30 | 8.2 |
| Dextromoramide tartrate | 1 in 25 | 7.1 |
| Hydrocodone bitartrate | 1 in 10 | 8.3 |
| Hydromorphone hydrochloride | 1 in 3 | 8.2 |
| Pethidine hydrochloride | very soluble | 8.7 |
| Methadone hydrochloride | 1 in 2 | 8.3 |
| Morphine sulphate | 1 in 21 | 8.0, 9.9 |
| Propoxyphene hydrochloride | 1 in 0.3 | |

In the following description the active ingredient will be illustrated by reference to the opioid drug morphine. However, this is illustrative only and the invention is in no way restricted thereto.

Morphine acts as an agonist primarily at mu, kappa and perhaps delta receptors in the central nervous system. By acting on these receptors the following pharmacological effects are seen. Analgesia due to a central action on pain perception, together with a modulatory effect on the central transmission of noxious sensation. It also causes drowsiness and euphoria (though sometimes dysphoria, particularly in those who are free of pain).

The pharmaceutical pellet composition according to the present invention may include a plurality of coated core elements.

The pharmaceutical composition may be provided in any suitable unit dosage form. An encapsulated form may be used.

The pharmaceutical pellet composition may be provided in a pellet or tableted pellet form. A tablet may be formed by compression of the pellets optionally with the addition of suitable excipients.

In a preferred aspect of the present invention the core coating, in use, generates a dissolution profile for the sustained release composition, which is equal to or greater than the minimum dissolution profile required to provide substantially equivalent bioavailability to a capsule, tablet or liquid containing an equal amount of the at last one active ingredient in an immediately available form.

"Dissolution profile" as used herein, means a plot of amount of active ingredient released as a function of time. The dissolution profile may be measured utilising the Drug Release Test (724) which incorporates standard test USPXXII 1990. (Test(711)). A profile is characterised by the test conditions selected. Thus the dissolution profile may be generated at a preselected shaft speed, temperature and pH of the dissolution media.

A first dissolution profile may be measured at a pH level approximating that of the stomach. At least a second dissolution profile may be measured at pH levels approximating that of at least one point in the intestine.

A highly acidic pH may simulate the stomach and a less acidic to basic pH may simulate the intestine. By the term "highly acidic pH" as used herein we mean a pH in the range of approximately 1 to 4. By the term "less acidic to basic pH" we mean a pH of greater than 4 up to approximately 7.5, preferably approximately 6 to 7.5.

A pH of approximately 1.2 may be used to simulate the pH of the stomach.

A pH of approximately 6.0 to 7.5 preferably 7.5 may be used to simulate the pH of the intestine.

Accordingly in a further preferred aspect, a first dissolution profile is measured at a pH level approximating that of the stomach and a second dissolution profile is measured at a pH level approximating that of at least one point in the intestine; the first and second dissolution profiles for the sustained release composition each being equal to or greater than the minimum dissolution required to provide substantially equivalent bioavailability to a capsule, tablet or liquid containing the at least one active ingredient in an immediately available form.

More preferably, the composition, in use, exhibits less fluctuations in plasma concentrations in active ingredient at steady state over a 24 hour period, relative to the active ingredient in an uncoated form and/or exhibits less diurnal variation in plasma concentration of active ingredient relative to known capsules or tablets containing the at least one active ingredient in a sustained release form.

For example, dissolution profiles have been generated which exhibit bioavailability substantially equivalent to, or better than, commercially known morphine compositions including MS Contin, MST Continus and morphine solution.

It will be understood that further since the active ingredient is provided in a sustained release pellet form significantly less fluctuations in plasma concentrations of active ingredients at steady state over a 24 hour period are encountered, and may allow for less frequent dosing relative to the active ingredient in an uncoated form. This is expected to result in less toxic and more effective therapeutic activity.

Similarly, it has been found that the pharmaceutical pellet composition according to the present invention exhibits less diurnal variation in plasma concentrations of active ingredient than prior art preparations, for example when administered on a two, three or four times daily dosage regimen.

Moreover, the pharmaceutical pellet composition according to the present invention shows no evidence of dose dumping. The relative bioavailability of the active ingredient generated from the pharmaceutical pellet composition is not compromised by food so that compliance will improve as the product may be taken without regard to meals.

Moreover, since the core coating is partially soluble at an acidic pH, for example as encountered in the stomach of the patients, some slow release of active ingredient will occur in the stomach. The slow rate of release of active ingredient may also be at a relatively constant rate.

The active ingredient may be available for absorption even in regions of the gastrointestinal tract which are not sufficiently alkaline to dissolve the enteric core coating component.

Thus the active ingredient is available for absorption in an absorption region substantially immediately after the pyloric sphincter in the patient. Such an absorption region may generally be characterised by a pH between approximately 1.2 and 5.5. Absorption will occur in the small intestine but since absorption will continue over an extended period of time, thus some absorption will occur additionally some way into the large intestine.

Where the active ingredient is a morphine compound, the morphine compound may take any suitable form. The morphine compound may be present in an anhydrous or hydrous form. The morphine compound may be provided in a salt form. Morphine sulphate is preferred. Morphine sulphate pentahydrate is particularly preferred.

Advantages of the sustained release pharmaceutical pellet composition according to the present invention may thus be summarised as follows
(i) The time during which morphine blood levels at steady state are greater than or equivalent to 75% of maximum blood levels (t>0.75Cₘₐₓ) may be approximately 3 hours or greater. Generally t>0.75Cₘₐₓ may be 3.5 hours or greater (t>0.75Cₘₐₓ for MS Contin has been reported to be is 3.5 hours).
(ii) peak to trough variations in blood morphine concentrations at steady state will be between 60-100% (these variations for MS Contin have been reported to be are approximately 300% and for Morphine Solution 4 hourly are approximately 200%)
(iii) diurnal variations may be reduced
(iv) the co-administration of food will not significantly decrease the extent of morphine absorption or alter the rate of morphine absorption when compared with administration in the fasted state the effect of food on morphine absorption from MS Contin is not known)
(v) inter- and intra-subject variation in blood morphine pharmacokinetics may be reduced.

The hybrid core coating may include
at least one polymer which is substantially insoluble independent of pH (insoluble matrix polymer); and
at least one enteric polymer which is substantially insoluble at acidic pH but at least partially soluble at a less acidic to basic pH (enteric polymer);
at least one component which is at least partially soluble at acidic pH (acid soluble polymer).

It has been found necessary in order to achieve a slow rate of release at acidic pH for pH dependent or independent drugs, and faster relatively constant rate of release over an extended period of time to include the above three components in the hybrid core coating composition.

Preferably the enteric polymer is readily soluble at a less acidic to basic pH.

Preferably the at least partially soluble component is a readily water-soluble component.

Accordingly the hybrid core coating may include an effective amount of
a matrix (insoluble) polymer which is substantially insoluble independent of pH
an enteric polymer whose solubility is pH dependent, and
an at least partially acid soluble component.

The rate of dissolution at highly acidic pH of the hybrid core coating will depend on the amount of the at least one partially acid soluble component, the pH dependent and pH independent polymers, and the thickness of the coating. Typical core coatings may be in the range of approximately 5 to 200 um, preferably approximately 25 to 50 um. It will be understood, accordingly, that the rate of absorption may be modified by modifying the thickness and/or the composition of the hybrid core coating.

Once a minimum amount of the at least partially acid soluble component and/or the maximum thickness of the coating to achieve the minimum dissolution profile at an highly acidic pH has been established, then it is simply a matter of design choice to adjust the composition and/or thickness of coating as desired.

It has been found that the dissolution rate of the soluble drug at various pH's can be modified at will by altering the ratio of polymers. The ternary system of polymers according to the present invention allows greater flexibility than as known in prior art using only binary systems of polymers.

The at least one enteric polymer may be selected from cellulose acetate phthalate, hydroxypropyl methylcellulose phthalate (HPMCP), polyvinyl acetate phthalate, methacrylic acid copolymer, hydroxypropyl methylcellulose acetate succinate, shellac, cellulose acetate trimellitate and mixtures thereof. Particularly preferred enteric polymers include synthetic resin bearing carboxyl groups. The methacrylic acid: acrylic acid ethylester 1:1 copolymer sold under the trade designation "Eudragit L100-55" has been found to be suitable.

The at least one enteric polymer may be present in the coating in an amount of from approximately 1 to 60% by weight, preferably 2 to 20% by weight, more preferably 5 to 15% by weight, based on the total weight of the hybrid core coating excluding weight of filler and plasticiser.

The at least partially acid-soluble component may be selected from polymers such as polyvinyl pyrrolidone, hydroxypropyl cellulose, hydroxypropyl methylcellulose, polyethylene glycol, polyvinyl alcohol and monomers therefor such as sugars, salts, or organic acids and mixtures thereof.

The at least partially acid-soluble component may be present in the coating in amounts of from approximately 1 to 60%, preferably 15 to 40% by weight, more preferably 20 to 35% by weight, based on the total weight of the hybrid core coating excluding weight of filler and plasticiser.

The at least one insoluble matrix polymer may be any suitable pharmaceutically acceptable polymer substantially insoluble independent of pH. The polymer may be selected from ethylcellulose, acrylic and/or methacrylic ester polymers or mixtures thereof and the like may be used. Polymers or copolymers of acrylates or methacrylates having a low quaternary ammonium content may be used. The acrylic acid ethyl ester: methacrylic acid methyl ester (1:1) copolymer has been found to be suitable.

The at least one insoluble matrix polymer may be present in the coating in an amount of from approximately 1 to 85% by weight preferably 35 to 75% by weight, more preferably 45 to 65% by weight, based on the total weight of the hybrid core coating excluding weight of filler and plasticiser.

The hybrid core coating may further include at least one plasticiser; and optionally
at least one filler.

Accordingly in a preferred aspect the hybrid core coating includes
0 to approximately 50% by weight, preferably 2.5 to 30% by weight, based on the total weight of the hybrid core coating of at least one plasticiser selected from diethyl phthalate, triethyl citrate, triethyl acetyl citrate, triacetin, tributyl citrate, polyethylene glycol and glycerol and the like; and
0 to approximately 75% by weight based on the total weight of the hybrid core coating of a filler selected from insoluble materials such as silicon dioxide, titanium dioxide, talc, alumina, starch, kaolin, polacrilin potassium, powdered cellulose, and microcrystalline cellulose and mixtures thereof.

The at least one plasticiser may be selected from diethyl phthalate, triethyl citrate, triethyl acetyl citrate, triacetin, tributyl citrate, polyethylene glycol and glycerol and the like. It will be understood that the plasticiser used may be largely dictated by the polymer used in the coating formulation, and the compatibility of the plasticiser with coating solution or dispersion. It should be noted that acid or water soluble plasticisers can also be used to function as the partially acid soluble component. The plasticiser may function to improve the physical stability of the core coating. A plasticiser is particularly preferred where the polymer has a high glass transition temperature and/or is of a relatively low molecular weight.

The plasticiser may be present in any suitable effective amount. Amounts of from approximately 0 to 50% by weight preferably 2.5 to 30% by weight based on the total weight of the hybrid core coating, have been found to be suitable.

The filler may be present in any suitable effective amount. Amounts of from 0 to approximately 75% by weight, preferably 15 to 60% by weight, more preferably 25 to 45% by weight, based on the total weight of the hybrid core coating have been found to be suitable.

Accordingly in a further preferred aspect the hybrid core coating has a formulation

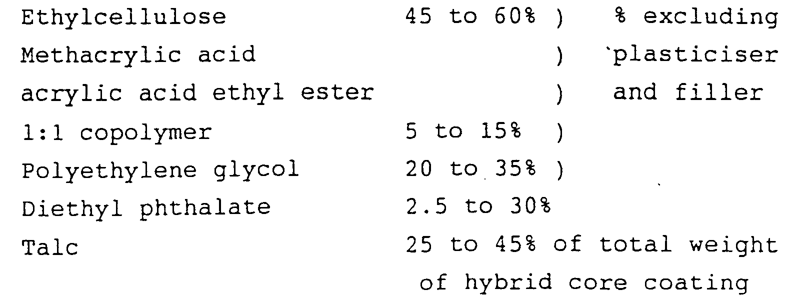

In a preferred aspect of the present invention the core element of the pharmaceutical composition according to the present invention may include an effective amount of
at least one active ingredient
at least one core seed; and
at least one binding agent.

The active ingredient may be present in any suitable effective amount. The amount of active ingredient is dependent on the potency of the active ingredient and on the desired dosage strength and volume of a unit dose of the drug product. The active ingredient may be present in amounts of approximately 0.1 to 95% by weight, based on the total weight of the core element. The active ingredient may preferably be a morphine compound. The morphine compound may be present in amounts of approximately 10 to 60% by weight, based on the total weight of the core element.

The binding agent may be present in amounts of from approximately 0.1 to 45% by weight preferably approximately 0.1 to 20% by weight based on the total weight of the core element.

The binding agent may be of any suitable type. Suitable binders may be selected from polyvinyl pyrrolidone, hydroxypropyl cellulose, hydroxypropyl methylcellulose, methylcellulose and hydroxyethyl cellulose, sugars and mixtures thereof. The binding agent may be provided in the form of a granulating solution. An aqueous or organic solvent may be included. Methanol, ethanol or mixtures thereof may be used as solvents.

The size and amount of the core seed may vary substantially from approximately 100 um to 1700 um depending upon the amount of active ingredient to be included. Accordingly, the core seeds may vary from approximately 5 to 99% by weight, preferably 40 to 90% by weight based on the total weight of the core element, depending on the potency of the active ingredient. The core seed may be of such a diameter to provide a final core element having a diameter of approximately 200 to 2000 um.

The core seed may be of any suitable type. A sugar or an active core seed may be used.

The core element may further include other carriers or excipients, fillers, stabilizing agents and colorants. Suitable fillers may be selected from insoluble materials such as silicon dioxide, talc, titanium dioxide, alumina, starch, kaolin, polacrilin potassium, powdered cellulose, and microcrystalline cellulose and mixtures thereof. Soluble fillers may be selected from mannitol, sucrose, lactose, dextrose, sodium chloride, sorbitol and mixtures thereof.

In a preferred aspect the core element includes an effective amount of at least one morphine compound; optionally
at least one core seed; and
at least one binding agent.

The core element may have a formulation
- Morphine sulphate: 10 to 60% by weight
- Core seeds: 30 to 89.9% by weight
- Hydroxypropylmethylcellulose: 0.1 to 10% by weight

Alternatively the core element may have a formulation
- Morphine sulphate: 10 to 60% by weight
- Core seeds: 30 to 87.5% by weight
- Polyvinyl pyrrolidone: 2.5 to 10% by weight

The hybrid core coating composition may be provided in the form of a solution, dispersion or suspension.

In the form of a solution, the solvent may be present in amounts of from approximately 25 to 97% by weight, preferably 85 - 97%, based on the total weight of the hybrid core coating composition. The solvent for the polymer may be a solvent such as water, methanol, ethanol, methylene chloride and mixtures thereof.

In the formof a dispension or suspension, the diluting medium may be present in amounts of from approximately 25 to 97% by weight, preferably 75 - 97%, based on the total weight of the hybrid core coating composition and is comprised predominantly of water.

Typical hybrid core coating formulations may be prepared in the amounts as follows:

### Core Coating Formulation

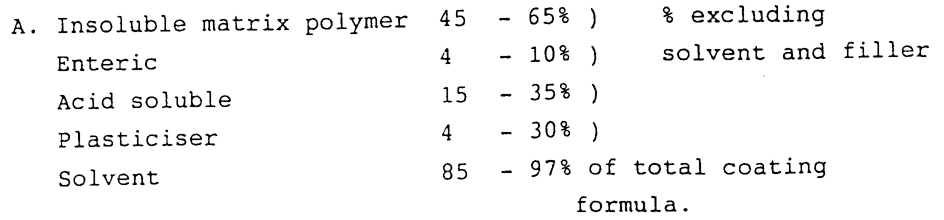

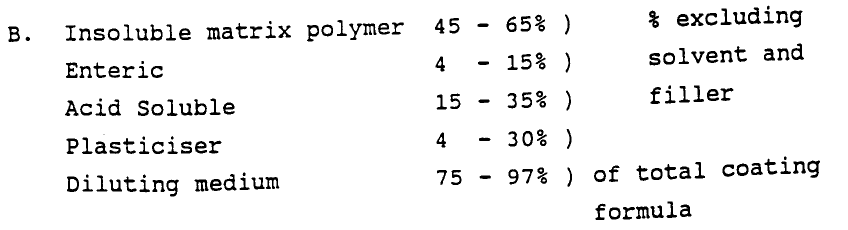

Optionally, an amount of filler not exceeding 50% of the core coating formulations weight exluding solvent, may be added.

The sustained release pharmaceutical composition of the present invention may be prepared by the following methods which includes
providing
a core element including
   at least one active ingredient which is an opiate agonist selected from the group consisting of the salts of codeine, dextromoramide, hydrocodone, hydromorphine, pethidine, methadone, morphine and propoxyphene; and
   at least one binding agent; and
a hybrid core coating composition including a solution, suspension or dispersion of
   at least one polymer which is substantially insoluble independent of pH;
   at least one enteric polymer which is substantially insoluble at acidic pH but at least partially soluble at a less acidic to basic pH; and
   at least one component which is at least partially soluble at acidic pH;
introducing the core element into a fluidised bed reactor; and
spraying the hybrid core coating composition onto the core element.

The method may further include the preliminary steps of
providing
   at least one active ingredient;
   at least one binding agent;
   at least one core seed; and
coating the core seeds with the active ingredient and binding agent to form a core element.

In an alternative form the at least one binding agent is provided in a granulating solution. In this form the coating step may be conducted as a spheronisation process. The spheronisation process includes contacting the core seeds with the active ingredient and simultaneously adding the granulating solution thereto. The spheronisation process may be conducted in a spheronising machine.

In a further alternative the method may further include the preliminary steps of
providing
   at least one active ingredient;
   at least one binding agent; and
   an effective amount of a solvent,
mixing the ingredients; and
subjecting the ingredients to an extrusion followed by marumerisation to form a core element.

The solvent may be an aqueous or organic solvent or mixtures thereof. The solvent may be present in an amount effective to allow the ingredients to be extruded.

The core elements formed are then subjected to a drying step. The drying step may be conducted in a fluidised bed or drying oven.

In a preferred form the at least one binding agent and active ingredient are provided in a solution or slurry. In this form the core seeds are sprayed with the solution or slurry. The spraying step may be conducted in any suitable coating equipment. The coating equipment may be a fluidised bed chamber, preferably a rotary fluid bed machine.

Spray coating of core elements may be undertaken utilising bottom, top or tangentially located spray nozzles. A bottom spray nozzle may reside proximate to the base of the fluidised bed facing upwards while a top spraying nozzle is located above the contents of the bed and facing downwards. The spray nozzle may reside in the mid-section of the fluidised bed and be oriented such as to spray tangentially to the rotating core elements.

The sustained release pharmaceutical pellet composition may be administered under a similar dosage regimen to that used in the prior art. The multi-pellet encapsulated form may for example be administered every eight to twenty-four hours in sustained release form.

In a further preferred aspect of the present invention the pharmaceutical pellet composition incorporating morphine compound may provide effective pain relief with twice or three times or four times daily administration. Versatility of dozing may be achieved with 10 mg, 20 mg, 50 mg, 100 mg, 200 mg, 500 mg or any other dose strength of capsules required.

The pharmaceutical pellet composition may be in multi-pellet encapsulated, sprinkle sachet or tableted forms.

In accordance with a further aspect of the present invention, there is provided a method of treating pain-associated conditions in patients requiring such treatment which method includes administering to a patient an effective amount of a sustained release pharmaceutical pellet composition including
a core element including at least one morphine compound of high solubility; and
a core coating for the core element which is partially soluble at a highly acidic pH and wherein the morphine compound is available for absorption at a relatively constant rate in the intestine over an extended period of time.

The method of treatment according to this aspect of the present invention is particularly applicable to the treatment of acute and chronic pain, particularly pain associated with terminal disease such as cancer and chronic backpain, as well as post-operative pain.

Preferably the pharmaceutical sustained release composition is provided in a unit dosage form and administration occurs at intervals of approximately 8 to 24 hours.

The present invention will now be more fully described with reference to the accompanying examples. It should be understood, however, that the following description is illustrative only and should not be taken in any way as a restriction on the generality of the invention specified above.

### EXAMPLE 1

### 1. Formulation 1

### Core Composition 1

| | |
|---|---|
| Morphine Sulphate pentahydrate | 194 g |
| Core seeds | 170 g |
| Polyvinyl pyrrolidone | 37 g |
| Ethanol | 185 g |

### Hybrid Core Coating Composition 1

| | |
|---|---|
| Polyethylene Glycol | 12 g |
| Ethylcellulose | 25 g |
| Diethyl phthalate | 2 g |
| Methacrylic acid : acrylic acid ethyl ester 1:1 copolymer | 5 g |
| Talc | 22 g |
| Ethanol | 667 g |

### 2. Formulation 2

### Core Composition 2

| | |
|---|---|
| Morphine Sulphate pentahydrate | 194 g |
| Core Seeds | 170 g |
| Polyvinyl pyrrolidone | 37 g |
| Ethanol | 185 g |

### Hybrid Core Coating Composition 2

| | |
|---|---|
| Polyethylene Glycol | 25 g |
| Ethylcellulose | 41 g |
| Diethyl phthalate | 3 g |
| Methacrylic acid: acrylic acid ethyl ester 1:1 copolymer | 4 g |
| Talc | 37 g |
| Ethanol | 1106 g |

### 3. Formulation 3

### Core Composition 3

| | |
|---|---|
| Morphine Sulphate Pentahydrate | 364 g |
| Core Seeds | 733 g |
| Hydroxypropylmethylcellulose | 14 g |
| Ethanol | 986 g |

### Hybrid Core Coating Composition 3

| | |
|---|---|
| Polyethylene Glycol | 47 g |
| Ethylcellulose | 90 g |
| Diethyl phthalate | 19 g |
| Methacrylic acid : acrylic acid ethyl ester 1:1 copolymer | 20 g |
| Talc | 88 g |
| Ethanol | 2509 g |

### Spheronised Core Manufacture (Core Composition 1 and 2)

The core seeds were placed in a spheroniser. The core seeds were then coated with a dry mixture of the active ingredients and inactive excipients whilst concomittantly adding a solution of the binder components.

The wet cores so formed were then dried in a fluidised bed dryer for 1 hour.

### Rotacoating Core Manufacture (Core Composition 3)

The core seeds were placed in a rotor fluid bed machine. The core seeds were then coated with a suspension or solution of the active ingredients and inactive excipients including at least one binding agent, in a suitable liquid. The wet cores so formed were then dried in a suitable drier for one hour.

### Pellet Manufacture

(a) The dried spheronised cores 1 and 2 were then placed in a fluid bed coating apparatus. The hybrid core coating compositions 1 and 2 were then sprayed onto the cores 1 and 2 to form Formulation 1 and 2 pellets respectively. At the conclusion of the process, the pellets were fluid bed dried.
(b) The dried cores 3 were then placed in a rotary fluid bed or conventional fluid bed coating apparatus. The hybrid core coating composition 3 was then sprayed onto the cores 3 to form Formulation 3 pellets.

A dissolution test was conducted on the pellet compositions 1, 2 and 3 utilising the test method USPXXII 1990 (Test 711). A sample is dissolved in an aqueous medium previously degassed and equilibrated to 37°C. The media are USP pH 1.2 media without enzymes and pH 7.5 phosphate buffer. A sample of known volume is withdrawn at designated time intervals from the bath as directed and subjected to a suitable assay procedure. The mg of morphine sulphate as a function of time is plotted as the dissolution profile.

The tests were conducted at pH 1.2 and pH 7.5.

The baskets containing the samples were rotated at approximately 50 r.p.m. and the aqueous medium maintained at approximately 37°C.

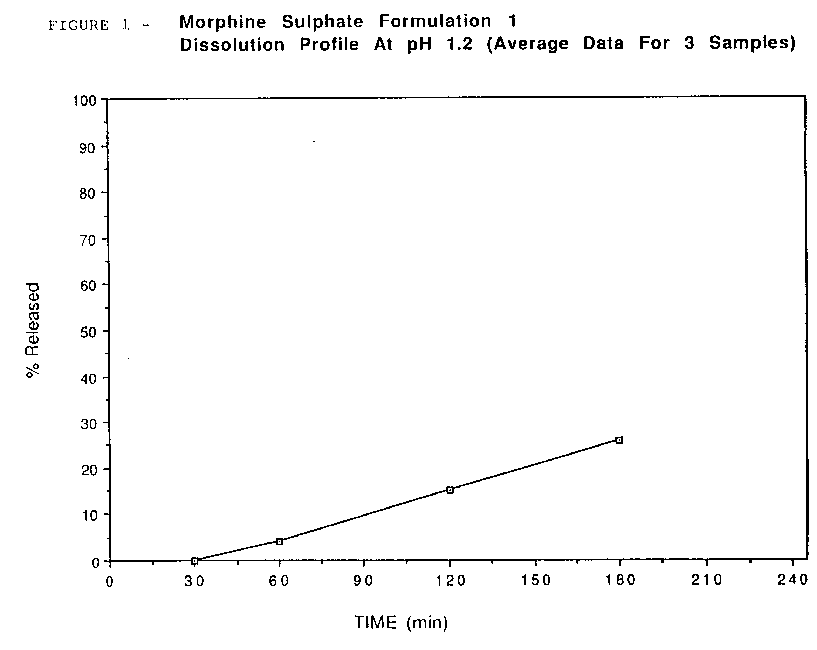

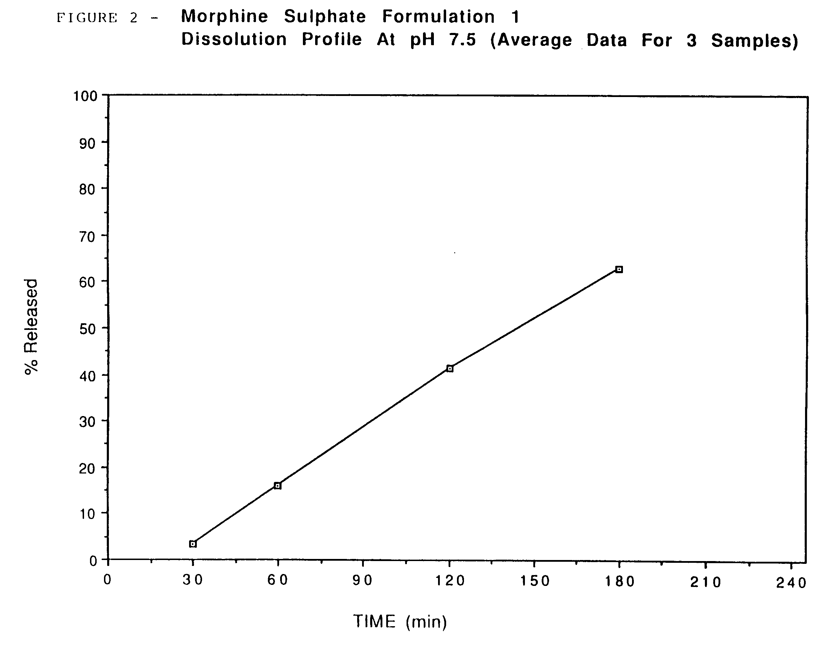

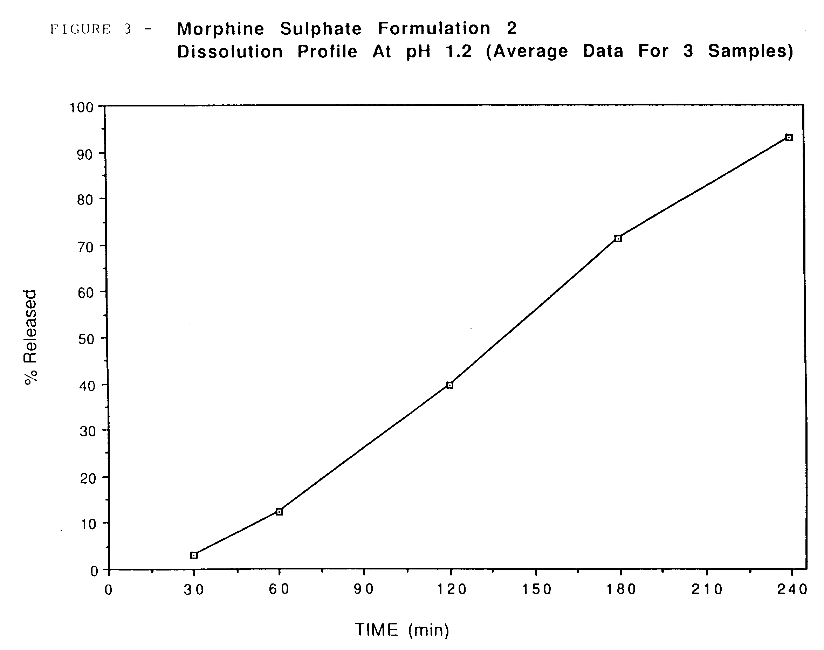

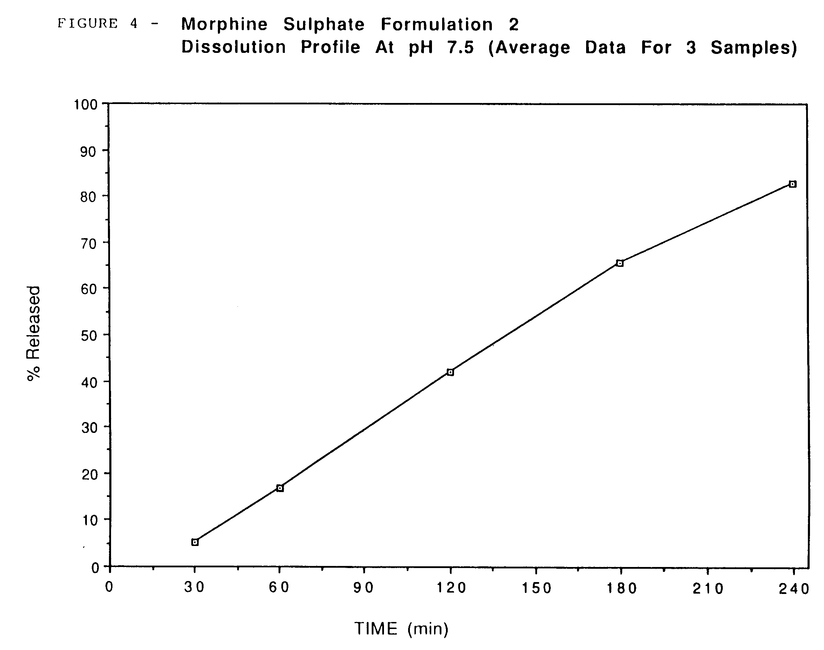

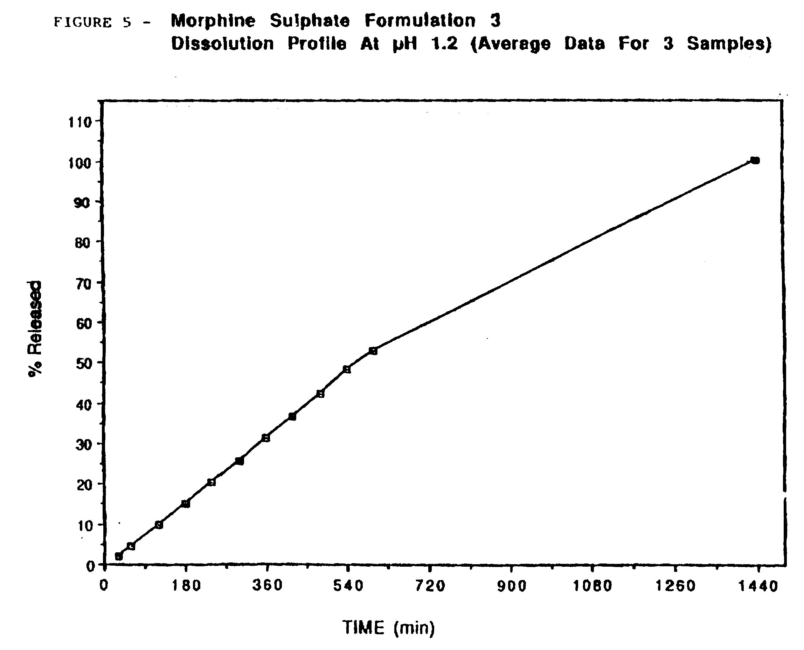

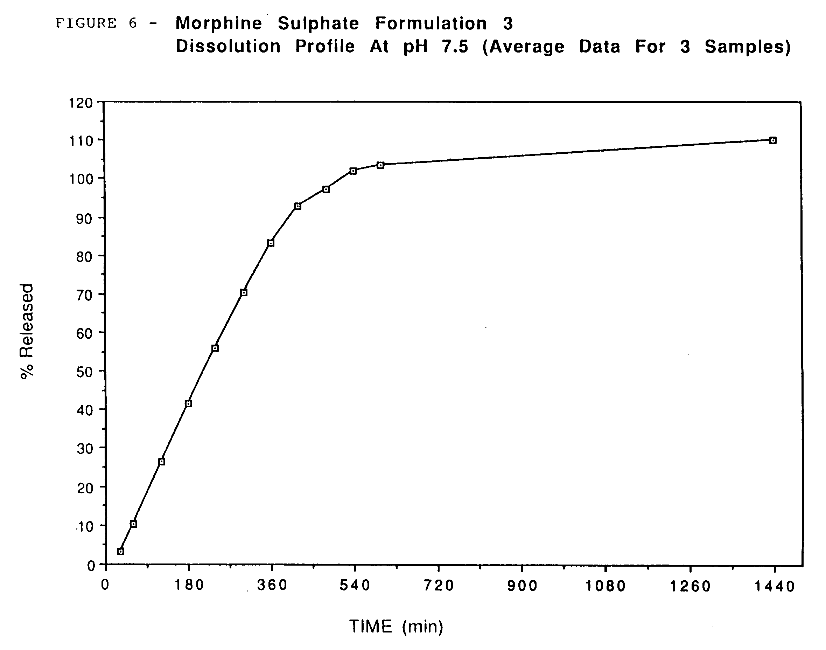

The results are given in Tables 1 to 6 and Figures 1 and 6 herein. The results for Formulation 1 at pH 1.2 and 7.5 are given in Tables 1 and 2 respectively. The hybrid coating on Formulation 1 pellet allows dissolution at pH 1.2, a significantly faster rate of dissolution is observed at pH 7.5. The results for Formulation 2 pellet at pH 1.2 and 7.5 are given in Tables 3 and 4 respectively, and are similar to those obtained from composition A.

The results for Formulation 3 pellets are similar to those achieved for Formulation 1 at pH 7.5. The results achieved for Formulation 3, however, illustrate a significant prolongation of release thereover.

**TABLE 1**

| DISSOLUTION DATA FOR FORMULATION 1 AT pH 1.2 (AVERAGED DATA FOR 3 SAMPLES) | | | | |
|---|---|---|---|---|
| TIME MIN. | MG RELEASED | SD | % RELEASED | SD |
| 30 | 0.00 | 0.00 | 0.00 | 0.00 |
| 60 | 2.29 | 0.09 | 4.04 | 0.15 |
| 120 | 8.43 | 0.18 | 14.88 | 0.28 |
| 180 | 14.66 | 0.39 | 25.87 | 0.71 |

**TABLE 2**

| DISSOLUTION DATA FOR FORMULATION 1 AT pH 7.5 (AVERAGED DATA FOR 3 SAMPLES) | | | | |
|---|---|---|---|---|
| TIME MIN. | MG RELEASED | SD | % RELEASED | SD |
| 30 | 1.85 | 0.09 | 3.28 | 0.17 |
| 60 | 9.03 | 0.25 | 16.07 | 0.45 |
| 120 | 23.20 | 0.42 | 41.29 | 0.77 |
| 180 | 35.39 | 0.50 | 63.00 | 1.01 |

**TABLE 3**

| DISSOLUTION DATA FOR FORMULATION 2 AT pH 1.2 (AVERAGED DATA FOR 3 SAMPLES) | | | | |
|---|---|---|---|---|
| TIME MIN. | MG RELEASED | SD | % RELEASED | SD |
| 30 | 1.64 | 0.00 | 3.22 | 0.01 |
| 60 | 6.26 | 0.09 | 12.25 | 0.16 |
| 120 | 20.24 | 0.18 | 39.63 | 0.46 |
| 180 | 36.39 | 0.27 | 71.27 | 0.72 |
| 240 | 47.47 | 0.49 | 92.97 | 1.12 |

**TABLE 4**

| DISSOLUTION DATA FOR FORMULATION 2 AT pH 7.5 (AVERAGED DATA FOR 3 SAMPLES) | | | | |
|---|---|---|---|---|
| TIME MIN. | MG RELEASED | SD | % RELEASED | SD |
| 30 | 2.63 | 0.00 | 5.12 | 0.03 |
| 60 | 8.69 | 0.09 | 16.94 | 0.11 |
| 120 | 21.62 | 0.33 | 42.13 | 0.40 |
| 180 | 33.66 | 0.59 | 65.60 | 0.79 |
| 240 | 42.47 | 0.82 | 82.78 | 1.13 |

**TABLE 5**

| DISSOLUTION DATA FOR FORMULATION 3 AT pH 1.2 (AVERAGED DATA FOR 3 SAMPLES) | | | | |
|---|---|---|---|---|
| TIME MIN. | MG RELEASED | SD | % RELEASED | SD |
| 30 | 1.44 | 0.39 | 2.12 | 0.53 |
| 60 | 3.03 | 0.33 | 4.48 | 0.39 |
| 120 | 6.78 | 0.30 | 10.03 | 0.36 |
| 180 | 10.17 | 0.18 | 15.04 | 0.34 |
| 240 | 13.87 | 0.41 | 20.51 | 0.29 |
| 300 | 17.45 | 0.31 | 25.81 | 0.30 |
| 360 | 21.29 | 0.21 | 31.49 | 0.27 |
| 420 | 24.75 | 0.32 | 36.62 | 0.46 |
| 480 | 28.60 | 0.64 | 42.30 | 0.37 |
| 540 | 32.63 | 0.42 | 48.28 | 0.45 |
| 600 | 35.80 | 0.92 | 52.95 | 0.37 |
| 24 hours | 67.60 | 1.26 | 100.04 | 3.79 |

**TABLE 6**

| DISSOLUTION DATA FOR FORMULATION 3 AT pH 7.5 (AVERAGED DATA FOR 3 SAMPLES) | | | | |
|---|---|---|---|---|
| TIME MIN. | MG RELEASED | SD | % RELEASED | SD |
| 30 | 2.19 | 0.11 | 3.23 | 0.17 |
| 60 | 7.05 | 0.89 | 10.38 | 1.26 |
| 120 | 18.07 | 1.05 | 26.63 | 1.44 |
| 180 | 28.12 | 1.03 | 41.44 | 1.35 |
| 240 | 37.86 | 1.05 | 55.80 | 1.32 |
| 300 | 47.60 | 1.48 | 70.16 | 1.96 |
| 360 | 56.33 | 0.54 | 83.03 | 0.47 |
| 420 | 63.03 | 2.01 | 92.90 | 2.76 |
| 480 | 65.97 | 0.61 | 97.23 | 0.75 |
| 540 | 69.13 | 0.41 | 101.89 | 0.79 |
| 600 | 70.20 | 0.43 | 103.47 | 0.45 |
| 24 hours | 74.76 | 2.36 | 110.19 | 3.04 |
| SD = Standard Deviation | | | | |

### EXAMPLE 2

Two sustained release morphine compositions in accordance with the present invention have been trialled in patients with back pain (fed and fasting) and in healthy volunteers (fasting). The results of these trials suggest that Faulding already has a product that is superior to a commercial product MS Contin with regard to sustained delivery of morphine. An investigation has also been initiated into understanding the effect that food has on the absorption of morphine.

The sustained release oral morphine compositions according to the present invention are designated Formulation 1 and Formulation 2.

### 1. PART A

A single dose 3 way crossover study under fasted conditions was conducted in six patients suffering chronic pain. On 3 occasions separated by one week, patients received a 50 mg oral morphine dose as either a solution (reference dose) or one of two sustained release formulations as pellets contained within a capsule (designated Formulation 1, a pH dependent release formulation; and Formulation 2, a pH independent release formulation). The doses were administered after an overnight fast. Venous blood samples were taken at specified time intervals from immediately after dose administration for 30 hours after the sustained release formulations and for 10 hours after the reference solution dose. The morphine concentration in the blood samples was quantitated using high pressure liquid chromatography (HPLC) with electrochemical detection. Table 3.1 summarizes the mean area under the curve (AUC); Cₘₐₓ (maximum blood concentration); Tₘₐₓ (time to reach peak blood concentration); T_{1/2} (apparent terminal half life); T_{≥0.75 Cmax} (time for which blood concentration was greater than 75% of Cₘₐₓ) and relative bioavailability (F%).

The results revealed that both Formulation 1 and Formulation 2 provide a sustained release relative to the reference solution as assessed by:
(1) a lower Cₘₐₓ for the formulations;
(2) a longer Tₘₐₓ for the formulations; and
(3) a longer time for which the blood morphine concentration was above 0.75 Cₘₐₓ for the formulations.

There was a significant decrease in Cₘₐₓ values for each formulation compared with the reference solution. The mean (±SD) Cₘₐₓ for the solution was 73.6±30.9 ng/mL whereas the corresponding values for Formulation 1 and Formulation 2 were 21.6±7.1 ng/mL and 23.2±4.8 ng/mL respectively. The variability in Cₘₐₓ for Formulations 1 and 2 as demonstrated by the coefficient of variation was significantly less than that of the solution in the same patients.

There was a significant increase in Tₘₐₓ values for the formulations relative to that obtained with the reference solution. The mean (±SD) Tₘₐₓ for solution was 1.07±1.09 hours whereas the equivalent values were 5.33±1.2 hours and 4.25±1.33 hours for Formulations 1 and 2 respectively. The variability in Tₘₐₓ values for the formulations was less than that obtained for the solution in the same patients.

The time the blood morphine concentration was greater than or equal to 0.75 Cₘₐₓ was significantly greater for the formulations compared to the reference solution dose. The mean time was 190 minutes for Formulation 1 and 237 minutes for Formulation 2 compared to only 59 minutes for the reference solution. Expressing these data as percentage of the time of the reference solution, Formulation 1 was 322% while Formulation 2 has 400% greater time that the blood morphine concentration was greater than 0.75 Cₘₐₓ compared to the solution.

There was no significant difference between the AUC for the formulations and that obtained for the reference solution (Table 3.1).

The relative bioavailability for the formulations was calculated from the ratio of the AUC for the appropriate formulation relative to that obtained for the reference solution for each patient. The relative bioavailability was 83.5% for Formulation 1 and 102.6% for Formulation 2.

The AUC and relative bioavailability data suggest that the extent of absorption of morphine from the three different formulations is similar whereas the Cₘₐₓ, Tₘₐₓ and T_{≥0.75Cmax} data indicate that the formulations exhibit the typical slower and prolonged absorption of a true sustained release preparation.

**TABLE 3.1**

| RESULT OF STUDY PART A | | | | | |
|---|---|---|---|---|---|
| PARAMETER | SOLUTION MEAN | FORMULATION 1 | | FORMULATION 2 | |
| | | MEAN | OBSERVED DIFF | MEAN | OBSERVED DIFF |
| AUC (ng.h/mL) | 199.77 | 170.72 | -29.05 | 193.77 | -6.0 |
| SD | ±66.32 | ±86.3 | | ±46.35 | |
| CV% | 33 | 51 | | 24 | |
| Cₘₐₓ (ng/mL) | 73.57 | 21.60 | -52.0 | 23.16 | -50.4 |
| SD | ±30.92 | ±7.12 | | ±4.76 | |
| CV% | 42 | 33 | | 21 | |
| Tₘₐₓ (hours) | 1.07 | 5.33 | 4.26 | 4.25 | 3.18 |
| SD | ±1.1 | ±1.21 | | ±1.33 | |
| CV% | 103 | 23 | | 31 | |
| Bioavailability (F%) | 100.0 | 83.53 | -16.47 | 102.62 | 2.62 |
| SD | ±0.00 | ±27.87 | | ±25.77 | |
| CV% | 0 | 33 | | 25 | |
| t_{1/2} (hours) | 3.02 | 6.58 | 3.56 | 7.65 | 4.63 |
| SD | ±1.97 | ±5.33 | | ±5.59 | |
| CV% | 65 | 81 | | 73 | |
| T_{≥0.75 Cmax} (minutes) | 59.0 | 189.8 | 130.8 | 237.3 | 178.3 |
| SD | ±37 | ±76 | | ±95 | |
| CV% | 63 | 40 | | 40 | |

### 2. PART B

A single dose 3 way crossover study under fed conditions was conducted in six patients suffering chronic pain. The same patients took part in both Parts A and B of this study. On 3 occasions separated by one week, patients received a 50 mg oral morphine dose as either a solution (reference dose) or one of two sustained release formulations as pellets contained within a capsule (designated Formulation 1, a pH dependent release formulation; and Formulation 2, a pH independent release formulation). The doses were administered after an overnight fast. Venous blood samples were taken at specified time intervals from immediately after dose administration for 30 hours after the sustained release formulations and for 10 hours after the reference solution dose. The morphine concentration in the blood samples was quantitated using high pressure liquid chromatography (HPLC) with electrochemical detection. Table 3.2 summarises the mean area under the curve (AUC); Cₘₐₓ (maximum blood concentration); Tₘₐₓ (time to reach peak blood concentration); T_{≥0.75 Cmax} (time for which blood concentration was greater than 75% of Cₘₐₓ) and relative bioavailability (F%).

The results revealed that, in the presence of food, both Formulation 1 and 2 provide a sustained release relative to the reference solution as assessed by:
(1) a lower Cₘₐₓ for the formulations;
(2) a longer Tₘₐₓ for the formulations; and
(3) a longer time for which the blood morphine concentration was above 0.75 Cₘₐₓ for the formulations.

There was a significant decrease in Cₘₐₓ values for each formulation compared with the reference solution. The mean (±SD) Cₘₐₓ for the solution was 80.7 ± 26.4 ng/mL whereas the corresponding values for Formulation 1 and Formulation 2 formulations were 22.0 ± 8.1 ng/mL and 32.6 ± 18.1 ng/mL respectively. The variability in Cₘₐₓ for Molly 1 and 2 as demonstrated by the coefficient of variation was similar for all formulations. The Cₘₐₓ values for each formulation obtained under fed conditions were similar to the values obtained in the same patients under fasting conditions

### (Part A).

There was a significant increase in Tₘₐₓ values for the formulations relative to that obtained with the reference solution. The mean (±SD) Tₘₐₓ for solution was 1.32 ± 1.65 hours whereas the equivalent values were 5.83 ± 0.75 and 4.5 ± 0.84 hours for Formulation 1 and 2 respectively. The variability in Tₘₐₓ values for the formulations was less than that obtained for the solution. The Tₘₐₓ values were similar under fed and fasted conditions for each respective formulation.

The time the blood morphine concentration was greater than or equal to 0.75 Cₘₐₓ was significantly greater for the formulations compared to the reference solution dose. The mean time was 231.2 minutes for Formulation 1 and 168.5 minutes for Formulation 2 compared to only 52.2 minutes for the reference solution. Expressing these data as percentage of the time of the reference solution, Formulation 1 was 443% while Formulation 2 has 323% greater time that the blood morphine concentration was greater than 0.75 Cₘₐₓ compared to the solution. The data for the time greater than 0.75 Cₘₐₓ under fed and fasting conditions was similar for each respective formulation.

Under fed conditions, there was a significant difference between the AUC for the formulations and that obtained for the reference solution (Table 3.2) the reference solution having a greater AUC than either formulation. The mean areas were very similar for the formulations with mean values of 204.13 ± 106.11 ng.h/mL and 225.09 ± 138.52 ng.h/mL for Formulation 1 and Formulation 2 respectively. The mean AUC for the solution under fed conditions was 281.98 ± 112.58 ng.h/mL. The intersubject variability in AUC was similar for all formulations as shown by the coefficient of variation.

A comparison of AUC data obtained under fed and fasted conditions shows that the AUC for the reference solution expressed as a ratio of fed/fasted was 1.41 (range 0.94 to 1.9) with all but one patient having a ratio of greater than unity. There was a similar trend with the Formulations in that the mean AUC obtained when the formulations were administered immediately after food were larger than the equivalent value obtained in the fasted state.

The primary concern was to establish that "dose dumping" did not occur with either formulation. The data indicate that the bioavailability of morphine from formulations in the presence of food is at least equivalent to and possibly greater than the bioavailability from the same formulation in the fasted state and that the formulations behave in a similar manner to the solution with regard to the influence of food on the absorption of morphine.

The relative bioavailability for the formulations relative to that obtained for the reference solution was 79.4% for Formulation 1 and 78.2% for Formulation 2.

The AUC and relative bioavailability data suggest that the extent of absorption of morphine from the formulations is similar but slightly less than the solution in the fed state whereas the Cₘₐₓ, Tₘₐₓ and T_{≥0.75 Cmax} data indicate that the formulations exhibit the typical slower and prolonged absorption of a true sustained release preparation.

**TABLE 3.2**

| RESULT OF STUDY PART B | | | | | |
|---|---|---|---|---|---|
| PARAMETER | SOLUTION MEAN | MOLLY 1 | | MOLLY 2 | |
| | | MEAN | OBSERVED DIFF | MEAN | OBSERVED DIFF |
| AUC (ng.h/mL) | 281.98 | 204.13 | -77.85 | 225.09 | -56.89 |
| SD | ±112.58 | ±106.11 | | ±138.52 | |
| CV% | 40 | 52 | | 62 | |
| Cₘₐₓ (ng/ml) | 80.66 | 22.00 | -58.66 | 32.63 | -48.03 |
| SD | ±26.44 | ±8.05 | | ±18.07 | |
| CV% | 33 | 37 | | 55 | |
| Tₘₐₓ (hours) | 1.32 | 5.83 | 4.51 | 4.50 | 3.18 |
| SD | ±1.65 | ±0.75 | | ±0.84 | |
| CV% | 125 | 13 | | 19 | |
| Bioavailability (F%) | 100.0 | 79.4 | -20.6 | 78.2 | -21.8 |
| SD | ±0.00 | ±47.3 | | ±27.1 | |
| CV% | 0 | 60.0 | | 35.0 | |
| T_{≥0.75Cmax} (minutes) | 52.2 | 231.2 | 179.0 | 168.5 | 116.3 |
| SD | ±39.3 | ±73.9 | | ±55.5 | |
| CV% | 75 | 32 | | 33 | |

## Claims (Claims for the following Contracting State(s): AT, BE, CH, DE, DK, FR, GB, IT, LI, LU, NL, SE)

1. A sustained release pharmaceutical pellet composition including
a core element including at least one active ingredient which is an opiate agonist selected from the group consisting of the salts of codeine, dextromoramide, hydrocodone, hydromorphine, pethidine, methadone, morphine and propoxyphene, and
a core coating for the core element which is partially soluble at a highly acidic pH, and including
a) at least 35% by weight of a matrix polymer which is insoluble independent of pH;
b) from 1 to 60% of an acid-soluble compound at a pH of from 1 to 4, sufficient to provide a slow rate of release of the active ingredient in the stomach; and
c) from 1 to 30% of an enteric polymer which is substantially insoluble at a pH of 1 to 4 but which is soluble at a pH of from 6 to 7.5;
the components in said coating being effective such that the active ingredient is available for absorption at a relatively constant rate of release in the intestine such that the composition delivers a therapeutically effective amount of said active ingredient over the course of a predetermined interval, so as to maintain an active ingredient blood level at steady state of at least 75% of maximum blood lever (t>0.75Cₘₐₓ) for approximately 3.5 hours or greater and so that the time at which the active ingredient reaches its maximum concentration (tₘₐₓ) is 4.5 hours or greater.

2. A sustained release pharmaceutical pellet composition according to claim 1 wherein the active ingredient is a morphine compound.

3. A sustained release pharmaceutical pellet composition according to claim 2, wherein the core coating, in use, is such as to provide substantially equivalent bioavailability as hereinbefore defined to a capsule, tablet or liquid containing an equal amount of the active ingredient in an immediate available form.

4. A sustained release pharmaceutical pellet composition according to claim 3 wherein the composition, in use, exhibits less fluctuations in plasma concentrations of active ingredient at steady state over a 24 hour period, relative to the active ingredient in an uncoated form and/or exhibits less diurnal variation in plasma concentration of active ingredient relative to prior art capsules or tablets containing the active ingredient in a sustained release form.

5. A sustained release pharmaceutical pellet composition according to any of claims 1 to 4 wherein the coating contains
as the matrix polymer (a), ethyl cellulose, acrylic ester polymers, methacrylic ester polymers, an acrylic acid ethyl ester : methacrylic acid methylester (1:1) copolymer, or a mixture thereof;
as acid-soluble compound (b), polyvinylpyrrolidone, hydroxypropyl cellulose, hydroxypropyl methylcellulose, polyethylene glycol, polyvinyl alcohol and monomers therefore and mixtures thereof; and
as the enteric polymer (c), cellulose acetate phthalate, hydroxypropyl methylcellulose phthalate, polyvinyl acetate phthalate, methacrylic acid : acrylic acid ethylester 1:1 copolymer, hydroxypropyl methylcellulose acetate succinate, shellac, cellulose acetate trimellitate and mixtures thereof.

6. A sustained release pharmaceutical pellet composition according to any of claims 1 to 5, wherein the coating comprises
35 to 75% by weight of component (a);
15 to 40% by weight of component (b); and
2 to 20% by weight of component (c).

7. A sustained release pharmaceutical pellet composition according to claim 6 wherein the coating also includes up to 50% of plasticiser selected from diethyl phthalate, triethyl citrate, triethyl acetyl citrate, triacetin, tributyl citrate, polyethylene glycol or glycerol and up to 75% of a filler, selected from silicon dioxide, titanium dioxide, talc, alumina, starch, kaolin, polacrilin potassium, powdered cellulose, and microcrystalline cellulose and mixtures thereof, said percentages being based on the total weight of the coating.

8. A sustained release pharmaceutical pellet composition according to claim 7 wherein the coating contains
component (a) 35 to 75%
component (b) 15 to 40%
component (c) 2 to 20%
plasticiser 2.5 to 30%

9. A sustained release pharmaceutical pellet composition according to any one of the preceding claims wherein the core element includes an effective amount of at least one active ingredient having an aqueous solubility of a least 1 in 30; at least one core seed, and at least one binding agent.

## Claims (Claims for the following Contracting State(s): ES, GR)

1. A method of producing a sustained release pharmaceutical pellet composition which comprises coating a core element, wherein the core element includes at least one active ingredient which is an opiate agonist selected from the group consisting of the salts of codeine, dextromoramide, hydrocodone, hydromorphine, pethidine, methadone, morphine and propoxyphene, with
a core coating which is partially soluble at a highly acidic pH, and which includes
a) at least 35% by weight of a matrix polymer which is insoluble independent of pH;
b) from 1 to 60% of an acid-soluble compound at a pH of from 1 to 4, sufficient to provide a slow rate of release of the active ingredient in the stomach; and
c) from 1 to 30% of an enteric polymer which is substantially insoluble at a pH of 1 to 4 but which is soluble at a pH of from 6 to 7.5;
the components in said coating being effective such that the active ingredient is available for absorption at a relatively constant rate of release in the intestine such that the composition delivers a therapeutically effective amount of said active ingredient over the course of a predetermined interval, so as to maintain an active ingredient blood level at steady state of at least 75% of maximum blood lever (t>0.75Cₘₐₓ) for approximately 3.5 hours or greater and so that the time at which the active ingredient reaches its maximum concentration (tₘₐₓ) is 4.5 hours or greater.

2. A method according to claim 1 wherein the active ingredient is a morphine compound.

3. A method according to claim 2, wherein the core coating, in use, is such as to provide substantially equivalent bioavailability as hereinbefore defined to a capsule, tablet or liquid containing an equal amount of the active ingredient in an immediate available form.

4. A method according to claim 3 wherein the composition, in use, exhibits less fluctuations in plasma concentrations of active ingredient at steady state over a 24 hour period, relative to the active ingredient in an uncoated form and/or exhibits less diurnal variation in plasma concentration of active ingredient relative to prior art capsules or tablets containing the active ingredient in a sustained release form.

5. A method according to any of claims 1 to 4 wherein the coating contains
as the matrix polymer (a), ethyl cellulose, acrylic ester polymers, methacrylic ester polymers, an acrylic acid ethyl ester : methacrylic acid methylester (1:1) copolymer, or a mixture thereof;
as acid-soluble compound (b), polyvinylpyrrolidone, hydroxypropyl cellulose, hydroxypropyl methylcellulose, polyethylene glycol, polyvinyl alcohol and monomers therefore and mixtures thereof; and
as the enteric polymer (c), cellulose acetate phthalate, hydroxypropyl methylcellulose phthalate, polyvinyl acetate phthalate, methacrylic acid : acrylic acid ethylester 1:1 copolymer, hydroxypropyl methylcellulose acetate succinate, shellac, cellulose acetate trimellitate and mixtures thereof.

6. A method according to any of claims 1 to 5, wherein the coating comprises:
35 to 75% by weight of component (a);
15 to 40% by weight of component (b); and
2 to 20% by weight of component (c).

7. A method according to claim 6 wherein the coating also includes up to 50% of plasticiser selected from diethyl phthalate, triethyl citrate, triethyl acetyl citrate, triacetin, tributyl citrate, polyethylene glycol or glycerol and up to 75% of a filler, selected from silicon dioxide, titanium dioxide, talc, alumina, starch, kaolin, polacrilin potassium, powdered cellulose, and microcrystalline cellulose and mixtures thereof, said percentages being based on the total weight of the coating.

8. A method according to claim 7 wherein the coating contains
component (a) 35 to 75%
component (b) 15 to 40%
component (c) 2 to 20%
plasticiser 2.5 to 30%

9. A method according to any one of the preceding claims wherein the core element includes an effective amount of at least one active ingredient having an aqueous solubility of a least 1 in 30; at least one core seed, and at least one binding agent.

## Patentansprüche (Patentansprüche für folgende(n) Vertragsstaat(en): AT, BE, CH, DE, DK, FR, GB, IT, LI, LU, NL, SE)

1. Arzneimittel mit Depotwirkung in Pillenform,
mit einem Kernelement, das wenigstens einen aktiven Inhaltsstoff enthält, der ein Opiatagonist ist, der aus einer Gruppe ausgewählt ist, zu der Salze von Codein, Dextromoramid, Hydrocodon, Hydromorphin, Pethidin, Methadon, Morphin und Propoxyphen gehören,
und mit einer Kernumhüllung für das Kernelement, die teilweise bei einem stark sauren pH-Wert löslich ist und enthält:
a) wenigstens 35 Gewichtsprozent eines Matrixpolymers, das unabhängig vom pH-Wert unlöslich ist;
b) 1 bis 60% einer säurelöslichen Verbindung, die bei einem pH-Wert zwischen 1 und 4 löslich ist und ausreicht, um zu einer langsamen Freigabegeschwindigkeit des wirksamen Inhaltsstoffes in dem Magen zu führen und
c) 1 bis 30% eines enteralen Polymers, das bei einem pH-Wert zwischen 1 und 4 im wesentlichen unlöslich, jedoch bei einem pH-Wert zwischen 6 und 7,5 löslich ist:
wobei die Komponenten in der Umhüllung derart wirken, daß der aktive Inhaltsstoff in dem Darm zur Absorption bei einer verhältnismäßig konstanten Freigaberate verfügbar ist, derart, daß das Arzneimittel eine therapeutisch wirksame Menge des aktiven Inhaltsstoffes über die Dauer eines vorbestimmten Intervalls zuführt, um so für näherungsweise 3,5 Stunden oder länger den Blutwert für den aktive Inhaltsstoff bei einem stationären Zustand von wenigstens 75% des maximalen Blutwertes (t>0,75Cₘₐₓ) zu halten, und derart, daß die Zeitspanne, während der der aktive Inhaltsstoff seine maximale Konzentration (tₘₐₓ) erreicht, 4,5 Stunden oder mehr beträgt.

2. Arzneimittel mit Depotwirkung in Pillenform nach Anspruch 1, wobei der aktive Inhaltsstoff eine Morphinverbindung ist.

3. Arzneimittel mit Depotwirkung in Pillenform nach Anspruch 2, wobei die Kernumhüllung sich bei der Verwendung derart verhält, daß sie eine im wesentlichen äquivalente Bioverfügbarkeit, wie sie oben definiert ist, für eine Kapsel, einer Tablette oder einer Flüssigkeit ergibt, die eine entsprechende Menge des aktiven Inhaltsstoffes in unmittelbar verfügbarer Form enthält.

4. Arzneimittel mit Depotwirkung in Pillenform nach Anspruch 3, wobei das Arzneimittel bei der Verwendung über eine 24-stündige Zeitperiode im stationären Zustand geringere Fluktuationen in den Plasmakonzentrationen des aktiven Inhaltsstoffes aufweist verglichen mit dem aktiven Inhaltsstoff bei einer unbeschichteten Form und/oder geringere diurnale Variationen bei der Plasmakonzentration des aktiven Inhaltsstoffes zeigt verglichen mit Kapseln oder Tabletten nach dem Stand der Technik, die den aktiven Inhaltsstoff in einer Depotform enthalten.

5. Arzneimittel mit Depotwirkung in Pillenform nach einem der Ansprüche 1 bis 4, wobei die Umhüllung für das Kernelement enthält:
als Matrix Polymer (a), Ethylzellulose, Acrylesterpolymere, Methacrylesterpolymere, Acrylsäureethylester: Methacrylsäure-Methylester-(1:1)-Copolymer, oder eine Mischung hiervon;
als säurelösliche Verbindung (b) Polyvinylpyrrolidon, Hydroxypropylzellulose, Hydroxypropylmethylzellulose, Polyethylenglykol, Polyvinylalkohol und Monomere hiervon sowie Mischungen daraus; und
als enterales Polymer (c) Zelluloseacetatphthalat, Hydroxypropylmethylzellulosephthalat, Polyvinylacetatphthalat, Methacrylsäure:Acrylsäureethylester-1:1-Copolymer, Hydroxypropyl-Methylzellulose-Acetatsuccinat, Schellack, Zellulose-Acetattrimellitat und Mischungen daraus.

6. Arzneimittel mit Depotwirkung in Pillenform nach einem der Ansprüche 1 bis 5, wobei die Kernumhüllung enthält:
35 bis 75 Gewichtsprozent der Komponente (a);
15 bis 40 Gewichtsprozent der Komponente (b) und
2 bis 20 Gewichtsprozent der Komponente (c).

7. Arzneimittel mit Depotwirkung in Pillenform nach Anspruch 6, wobei die Umhüllung außerdem bis zu 50% Plastifizierer, der aus einer Gruppe ausgewählt ist, zu der Diethylphthalat, Triethylcitrat, Triethylacetylcitrat, Triacetin, Tributylcitrat, Polyethylenglykol oder Glyzerol gehören, sowie bis zu 75% eines Füllers enthält, der aus einer Gruppe ausgewählt ist, zu der Siliziumdioxid, Titandioxid, Talcum, Aluminiumoxid, Stärke, Kaolin, Polyacrilinkalium, pulverförmige Zellulose und Mikrokristallinzellulose sowie Mischungen daraus gehören, wobei die Prozentangaben auf das Gesamtgewicht der Umhüllung bezogen sind.

8. Arzneimittel mit Depotwirkung in Pillenform nach Anspruch 7, wobei die Umhüllung enthält:
Komponente (a) 35 bis 75%
Komponente (b) 15 bis 40%
Komponente (c) 2 bis 20%
Plastifizierer 2,5 bis 30%.

9. Arzneimittel mit Depotwirkung in Pillenform nach einem der vorhergehenden Ansprüche, wobei das Kernelement eine wirksame Menge wenigstens eines aktiven Inhaltsstoffes mit einer Löslichkeit in Wasser von wenigstens 1 in 30; wenigstens einen Kernkeim und wenigstens ein Bindemittel enthält.

## Patentansprüche (Patentansprüche für folgende(n) Vertragsstaat(en): ES, GR)

1. Verfahren zum Herstellen eines Arzneimittels mit Depotwirkung in Pillenform, zu dem es gehört, daß ein Kernelement, das wenigstens einen aktiven Inhaltsstoff enthält, der ein Opiatagonist ist, der aus einer Gruppe ausgewählt ist, zu der Salze von Codein, Dextromoramid, Hydrocodon, Hydromorphin, Pethidin, Methadon, Morphin und Propoxyphen gehören,
mit einer Kernumhüllung umhüllt wird, die teilweise bei einem stark sauren pH-Wert löslich ist und enthält:
a) wenigstens 35 Gewichtsprozent eines Matrixpolymers, das unabhängig vom pH-Wert unlöslich ist;
b) 1 bis 60% einer säurelöslichen Verbindung, die bei einem pH-Wert zwischen 1 und 4 löslich ist und ausreicht, um zu einer langsamen Freigabegeschwindigkeit des wirksamen Inhaltsstoffes in dem Magen zu führen und
c) 1 bis 30% eines enteralen Polymers, das bei einem pH-Wert zwischen 1 und 4 im wesentlichen unlöslich, jedoch bei einem pH-Wert zwischen 6 und 7,5 löslich ist:
wobei die Komponenten in der Umhüllung derart wirken, daß der aktive Inhaltsstoff in dem Darm zur Absorption bei einer verhältnismäßig konstanten Freigaberate verfügbar ist, derart, daß das Arzneimittel eine therapeutisch wirksame Menge des aktiven Inhaltsstoffes über die Dauer eines vorbestimmten Intervalls zuführt, um so für näherungsweise 3,5 Stunden oder länger den Blutwert für den aktive Inhaltsstoff bei einem stationären Zustand von wenigstens 75% des maximalen Blutwertes (t>0,75Cₘₐₓ) zu halten, und derart, daß die Zeitspanne, während der der aktive Inhaltsstoff seine maximale Konzentration (tₘₐₓ) erreicht, 4,5 Stunden oder mehr beträgt.

2. Verfahren nach Anspruch 1, wobei der aktive Inhaltsstoff eine Morphinverbindung ist.

3. Verfahren nach Anspruch 2, wobei die Kernumhüllung sich bei der Verwendung derart verhält, daß sie eine im wesentlichen äquivalente Bioverfügbarkeit, wie sie oben definiert ist, für eine Kapsel, einer Tablette oder einer Flüssigkeit ergibt, die eine entsprechende Menge des aktiven Inhaltsstoffes in unmittelbar verfügbarer Form enthält.

4. Verfahren nach Anspruch 3, wobei das Arzneimittel bei der Verwendung über eine 24-stündige Zeitperiode im stationären Zustand geringere Fluktuationen in den Plasmakonzentrationen des aktiven Inhaltsstoffes aufweist verglichen mit dem aktiven Inhaltsstoff bei einer unbeschichteten Form und/oder geringere diurnale Variationen bei der Plasmakonzentration des aktiven Inhaltsstoffes zeigt verglichen mit Kapseln oder Tabletten nach dem Stand der Technik, die den aktiven Inhaltsstoff in einer Depotform enthalten.

5. Verfahren nach einem der Ansprüche 1 bis 4, wobei die Umhüllung für das Kernelement enthält:
als Matrix Polymer (a), Ethylzellulose, Acrylesterpolymere, Methacrylesterpolymere, Acrylsäureethylester: Methacrylsäure-Methylester-(1:1)-Copolymer, oder eine Mischung hiervon;
als säurelösliche Verbindung (b) Polyvinylpyrrolidon, Hydroxypropylzellulose, Hydroxypropylmethylzellulose, Polyethylenglykol, Polyvinylalkohol und Monomere hiervon sowie Mischungen daraus; und
als enterales Polymer (c) Zelluloseacetatphthalat, Hydroxypropylmethylzellulosephthalat, Polyvinylacetatphthalat, Methacrylsäure:Acrylsäureethylester-1:1-Copolymer, Hydroxypropyl-Methylzellulose-Acetatsuccinat, Schellack, Zellulose-Acetattrimellitat und Mischungen daraus.

6. Verfahren nach einem der Ansprüche 1 bis 5, wobei die Kernumhüllung enthält:
35 bis 75 Gewichtsprozent der Komponente (a);
15 bis 40 Gewichtsprozent der Komponente (b) und
2 bis 20 Gewichtsprozent der Komponente (c).

7. Verfahren nach Anspruch 6, wobei die Umhüllung außerdem bis zu 50% Plastifizierer, der aus einer Gruppe ausgewählt ist, zu der Diethylphthalat, Triethylcitrat, Triethylacetylcitrat, Triacetin, Tributylcitrat, Polyethylenglykol oder Glyzerol gehören, sowie bis zu 75% eines Füllers enthält, der aus einer Gruppe ausgewählt ist, zu der Siliziumdioxid, Titandioxid, Talcum, Aluminiumoxid, Stärke, Kaolin, Polyacrilinkalium, pulverförmige Zellulose und Mikrokristallinzellulose sowie Mischungen daraus gehören, wobei die Prozentangaben auf das Gesamtgewicht der Umhüllung bezogen sind.

8. Verfahren nach Anspruch 7, wobei die Umhüllung enthält:
Komponente (a) 35 bis 75%
Komponente (b) 15 bis 40%
Komponente (c) 2 bis 20%
Plastifizierer 2,5 bis 30%.

9. Verfahren nach einem der vorhergehenden Ansprüche, wobei das Kernelement eine wirksame Menge wenigstens eines aktiven Inhaltsstoffes mit einer Löslichkeit in Wasser von wenigstens 1 in 30; wenigstens einen Kernkeim und wenigstens ein Bindemittel enthält.

## Revendications (Revendications pour l'(les) Etat(s) contractant(s) suivant(s): AT, BE, CH, DE, DK, FR, GB, IT, LI, LU, NL, SE)

1. Composition pharmaceutique en pastilles à libération prolongée, comprenant :
une partie formant noyau contenant au moins un ingrédient actif consistant en un agoniste opiacé choisi parmi les sels de codéine, de dextromoramide, d'hydrocodone, d'hydromorphine, de péthidine, de méthadone, de morphine et de propoxyphène, et
un enrobage de noyau pour la partie formant noyau, partiellement soluble à un pH très acide, et contenant :
a) au moins 35 % en poids d'un polymère formant matrice insoluble indépendamment du pH ;
b) de 1 à 60 % d'un composé soluble dans un acide à un pH de 1 à 4 suffisant pour obtenir une vitesse de libération lente de l'ingrédient actif dans l'estomac ; et
c) de 1 à 30 % d'un polymère entérosoluble, à peu près insoluble à un pH de 1 à 4, mais soluble à un pH de 6 à 7,5 ;
les composants présents dans cet enrobage étant efficaces de telle façon que l'ingrédient actif soit disponible pour être absorbé à une vitesse de libération relativement constante dans l'intestin, de sorte que la composition fournisse une quantité thérapeutiquement efficace de cet ingrédient actif au cours d'un intervalle prédéterminé, de façon à maintenir une teneur sanguine en ingrédient actif à l'état stationnaire, d'au moins 75 % de la teneur sanguine maximum (t>0,75Cₘₐₓ) pendant environ 3,5 heures ou plus, et de telle façon que le moment où l'ingrédient actif atteint sa concentration maximum (tₘₐₓ) soit de 4,5 heures ou plus.

2. Composition pharmaceutique en pastilles à libération prolongée selon la revendication 1, dans laquelle l'ingrédient actif est un composé morphinique.

3. Composition pharmaceutique en pastilles à libération prolongée selon la revendication 2, dans laquelle l'enrobage de noyau est, en utilisation, approprié pour procurer une biodisponibilité à peu près équivalente, ainsi que cela a été défini précédemment, à une gélule, un comprimé ou un liquide contenant une quantité égale de l'ingrédient actif sous une forme immédiatement disponible.

4. Composition pharmaceutique en pastilles à libération prolongée selon la revendication 3, dans laquelle la composition donne lieu, en utilisation, à moins de fluctuations des concentrations plasmatiques d'ingrédient actif à l'état stationnaire pendant une période de 24 h, par rapport à l'ingrédient actif sous forme non enrobée et/ou à une moindre variation diurne de la concentration plasmatique en ingrédient actif par rapport aux gélules ou aux comprimés de l'art antérieur contenant l'ingrédient actif sous une forme à libération prolongée.

5. Composition pharmaceutique en pastilles à libération prolongée selon l'une quelconque des revendications 1 à 4, dans laquelle l'enrobage contient :
comme polymère formant matrice (a), de l'éthyl cellulose, des polymères d'ester acrylique, des polymères d'ester méthacrylique, un copolymère (1:1) d'ester éthylique d'acide acrylique et d'ester méthylique d'acide méthacrylique, ou des mélanges de ceux-ci ;
comme composé soluble dans un acide (b), de la polyvinylpyrrolidone, de l'hydroxypropyl cellulose, de l'hydroxypropyl méthylcellulose, du polyéthylène glycol, du poly(alcool vinylique), ainsi que des monomères et des mélanges de ceux-ci ; et
comme polymère entérosoluble (c), de l'acétate-phtalate de cellulose, du phtalate d'hydroxypropyl méthylcellulose, du poly(acétate-phtalate de vinyle), un copolymère 1:1 d'acide méthacrylique et d'ester éthylique d'acide acrylique, de l'acétate-succinate d'hydroxypropyl méthylcellulose, de la gomme-laque, de l'acétate-trimellate de cellulose et des mélanges de ceux-ci.

6. Composition pharmaceutique en pastilles à libération prolongée selon l'une quelconque des revendications 1 à 5, dans laquelle l'enrobage comprend :
de 35 à 75 % en poids du composant (a) ;
de 15 à 40 % en poids du composant (b) ; et
de 2 à 20 % en poids du composant (c).

7. Composition pharmaceutique en pastilles à libération prolongée selon la revendication 6, dans laquelle l'enrobage contient également jusqu'à 50 % d'un plastifiant choisi parmi le phtalate de diéthyle, le citrate de triéthyle, l'acétyl citrate de triéthyle, la triacétine, le citrate de tributyle, le polyéthylène glycol ou le glycérol, et jusqu'à 75 % d'une charge choisie parmi le dioxyde de silicium, le dioxyde de titane, le talc, l'alumine, l'amidon, le kaolin, la polacriline potassique, la cellulose pulvérisée, ainsi que la cellulose microcristalline et leurs mélanges, ces pourcentages étant basés sur le poids total de l'enrobage.

8. Composition pharmaceutique en pastilles à libération prolongée selon la revendication 7, dans laquelle l'enrobage contient :
composant (a) 35 à 75 %
composant (b) 15 à 40 %
composant (c) 2 à 20 %
plastifiant 2,5 à 30 %

9. Composition pharmaceutique en pastilles à libération prolongée selon l'une quelconque des revendications précédentes, dans laquelle la partie formant noyau comprend une quantité efficace d'au moins un ingrédient actif ayant une solubilité en milieu aqueux d'au moins 1 pour 30 ; au moins un germe de noyau, et au moins un agent de liaison.

## Revendications (Revendications pour l'(les) Etat(s) contractant(s) suivant(s): ES, GR)

1. Procédé de production d'une composition pharmaceutique en pastilles à libération prolongée, selon lequel on enrobe une partie formant noyau, la partie formant noyau comprenant au moins un ingrédient actif consistant en un agoniste opiacé choisi parmi les sels de codéine, de dextromoramide, d'hydrocodone, d'hydromorphine, de péthidine, de méthadone, de morphine et de propoxyphène, avec
un enrobage de noyau partiellement soluble à un pH très acide, et comprenant :
a) au moins 35 % en poids d'un polymère formant matrice insoluble indépendamment du pH ;
b) de 1 à 60 % d'un composé soluble dans un acide à un pH de 1 à 4 suffisant pour obtenir une vitesse de libération lente de l'ingrédient actif dans l'estomac ; et
c) de 1 à 30 % d'un polymère entérosoluble, à peu près insoluble à un pH de 1 à 4, mais soluble à un pH de 6 à 7,5 ;
les composants présents dans cet enrobage étant efficaces de telle façon que l'ingrédient actif soit disponible pour être absorbé à une vitesse de libération relativement constante dans l'intestin, de sorte que la composition fournisse une quantité thérapeutiquement efficace de cet ingrédient actif au cours d'un intervalle prédéterminé, de façon à maintenir une teneur sanguine en ingrédient actif à l'état stationnaire, d'au moins 75 % de la teneur sanguine maximum (t>0,75Cₘₐₓ) pendant environ 3,5 heures ou plus, et de telle façon que le moment auquel l'ingrédient actif atteint sa concentration maximum (tₘₐₓ) soit de 4,5 h ou plus.

2. Procédé selon la revendication 1, dans lequel l'ingrédient actif est un composé morphinique.

3. Procédé selon la revendication 2, dans lequel l'enrobage de noyau est, en utilisation, approprié pour procurer une biodisponibilité à peu près équivalente, ainsi que cela a été précédemment défini, à une gélule, un comprimé ou un liquide contenant une quantité égale de l'ingrédient actif sous une forme immédiatement disponible.

4. Procédé selon la revendication 3, dans lequel la composition donne lieu, en utilisation, à moins de fluctuations de concentrations plasmatiques en ingrédient actif à l'état stationnaire sur une période de 24 h, par rapport à l'ingrédient actif sous forme non enrobée et/ou à une moindre variation diurne de concentration plasmatique en ingrédient actif par rapport à des gélules ou des comprimés de l'art antérieur contenant l'ingrédient actif sous forme à libération prolongée.

5. Procédé selon l'une quelconque des revendications 1 à 4, dans lequel l'enrobage contient :
comme polymère formant matrice (a), de l'éthyl cellulose, des polymères d'ester acrylique, des polymères d'ester méthacrylique, un copolymère (1:1) d'ester éthylique d'acide acrylique et d'ester méthylique d'acide méthacrylique, ou un mélange de ceux-ci ;
comme composé soluble dans un acide (b), de la polyvinylpyrrolidone, de l'hydroxypropyl cellulose, de l'hydroxypropyl méthylcellulose, du polyéthylène glycol, du poly (alcool vinylique), ainsi que des monomères et des mélanges de ceux-ci ; et
comme polymère entérosoluble (c), de l'acétate-phtalate de cellulose, du phtalate d'hydroxypropyl méthylcellulose, du poly(acétate-phtalate de vinyle), un copolymère 1:1 d'acide méthacrylique et d'ester éthylique d'acide acrylique, de l'acétate-succinate d'hydroxypropyl méthylcellulose, de la gomme-laque, de l'acétate-trimellate de cellulose et des mélanges de ceux-ci.

6. Procédé selon l'une quelconque des revendications 1 à 5, dans lequel l'enrobage comprend :
de 35 à 75 % en poids du composant (a) ;
de 15 à 40 % en poids du composant (b) ; et
de 2 à 20 % en poids du composant (c).

7. Procédé selon la revendication 6, dans lequel l'enrobage contient également jusqu'à 50 % d'un plastifiant choisi parmi le phtalate de diéthyle, le citrate de triéthyle, l'acétyl citrate de triéthyle, la triacétine, le citrate de tributyle, le polyéthylène glycol ou le glycérol, et jusqu'à 75 % d'une charge choisie parmie le dioxyde de silicium, le dioxyde de titane, le talc, l'alumine, l'amidon, le kaolin, la polacriline potassique, la cellulose pulvérisée et la cellulose microcristalline ainsi que leurs mélanges, ces pourcentages étant basés sur le poids total de l'enrobage.

8. Procédé selon la revendication 7, dans lequel l'enrobage contient :
composant (a) 35 à 75 %
composant (b) 15 à 40 %
composant (c) 2 à 20 %
plastifiant 2,5 à 30 %

9. Procédé selon l'une quelconque des revendications précédentes, dans lequel la partie formant noyau comprend une quantité efficace d'au moins un ingrédient actif ayant une solubilité en milieu aqueux d'au moins 1 pour 30 ; au moins un germe de noyau, et au moins un agent de liaison.
